(19) Europäisches Patentamt
European Patent Office
Office européen des brevets

(11) Publication number: 0 497 448 A1

# (12) EUROPEAN PATENT APPLICATION

(21) Application number: 92300077.2

(51) Int. Cl.5: C12Q 1/68, G01N 27/26

(22) Date of filing: 06.01.92

(30) Priority: 01.02.91 US 649398

(43) Date of publication of application:
05.08.92 Bulletin 92/32

(84) Designated Contracting States:
CH DE FR GB IT LI SE

(71) Applicant: BECKMAN INSTRUMENTS, INC.
2500 Harbor Boulevard Box 3100
Fullerton California 92634-3100(US)

(72) Inventor: Konrad, Kenneth D.
3706 E. 5th Street
Long Beach, California 90814(US)
Inventor: Pentoney Jr., Stephen Lyle
5126 Lakeview Avenue
Yorba Linda, California 92686(US)

(74) Representative: Ede, Eric et al
Fitzpatricks 4 West Regent Street
Glasgow G2 1RS(GB)

## (54) Capillary gel electrophoresis in the presence of at least one neutral non-urea denaturing agent.

(57) Capillary gels and methods useful in the analysis of nucleic acid sequences are disclosed. The gels contain at least one neutral non-urea denaturing agent comprising at least one moiety capable of disrupting hydrogen bonding and at least one moiety capable of reducing intramolecular base pairing of nucleic acids. Preferably, the non-urea denaturing agent is an amide, where the hydrogen disrupting moiety is an amine, and the moiety capable of reducing intramolecular base pairing is selected from the group consisting of H, an alkyl having from between about 1 and about 6 carbon atoms, benzene, and benzene derivatives. Most preferably, the non-urea denaturing agent is formamide.

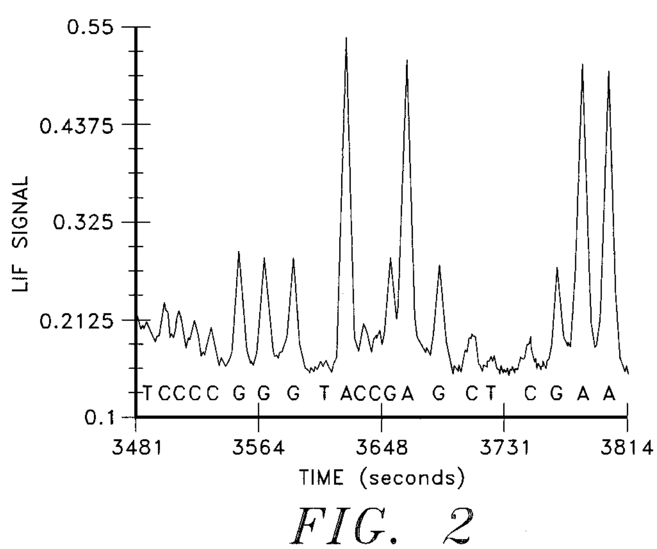


FIG. 2

EP 0 497 448 A1

## FIELD OF THE INVENTION

The present invention is related to the analysis of nucleic acid sequences by capillary electrophoresis, and specifically to gels useful in the analysis of nucleic acid sequences by capillary electrophoresis.

## BACKGROUND OF THE INVENTION

Articles set forth in the Background of the Invention are incorporated herein by reference.

Methods for sequencing nucleic acids have been described in the literature including two well known methods which rely on slab gel electrophoresis. Briefly, the Sanger-Colson method determines the sequence of DNA by incorporating radioactive chain-terminators into a synthesized oligonucleotide of specific nucleotide residues. Separate terminators are used for each of the four nucleotides (adenine, guanine, cytosine and thymine). A pattern of radioactive bands on a resulting autoradiography is then obtained from which the sequence can be determined.

The Maxam-Gilbert method involves the radioactive labelling of a DNA molecule at either the 5' or 3' terminus thereof. This is followed by breaking the DNA fragment at one of the nucleotides with a chemical agent. This, in turn, produces a radioactive fragment extending from the labelled end to the position of chemical cleavage. By using nucleotide-specific chemical agents for each of the nucleotides, the products of the four reactions can be resolved and fractionated according to size by electrophoresis on a polyacrylamide gel. The DNA sequence is then determined from a pattern of radioactive bands which appear on a resulting autoradiogram.

Automated DNA sequencers based upon slab gel electrophoresis techniques have, at best, a theoretical capacity of about 10,000 bases of raw sequence data per day. See, Smith, L. M. Amer. Biotech Lab, 7(5):10-25 (1989). The principal limitation to increased throughput is the relatively small magnitude of the electric field which can be applied to slab gels. While an increased magnitude of the electric field will theoretically increase separation speed, it will also promote excessive heat production. Excessive heat causes both decreased resolution and the breakdown of the gel material itself, two deleterious effects which must be avoided.

Capillary electrophoresis is an answer to the inherent problems of DNA sequencing using slab gel electrophoresis. Capillary electrophoresis ("CE") involves introduction of a sample into one end of a capillary tube and the application of an electric field to cause the sample to migrate toward the other end of the capillary. Because each of the constituents of the sample has its own distinct electrokinetic mobility in the gel matrix, they each travel through the capillary tube at different rates. This resolves the sample into discrete zones during migration through the capillary tube. An on-line detector can be used to continuously monitor the separation and provide data as to the various constituents based upon the discrete zones. The extremely small diameter of the capillaries (50-100$\mu$m i.d.) effectively removes heat caused by the applied electric field. Effective heat removal allows for greater electric fields and, consequently, higher resolution and throughput than is possible by slab gel electrophoresis. Recently, it has been demonstrated that capillary electrophoresis techniques can be utilized for the analyses of nucleic acid sequences with up to a factor of 25 times the speed of conventional slab electrophoresis. See Drossman, H. et al, "High Speed Separations of DNA Sequencing Reactions by Capillary Electrophoresis." Anal. Chem. 62: 900-903 (1990), and Smith, L. M. "DNA Sequence Analysis: Past, Present, and Future." Am. Biotechnol. Lab., 7(5): 10-25 (1989).

A problem associated with CE electrophoretic separation of single stranded nucleic acids is the formation of secondary (and unwanted) structures through intramolecular base pairing. The sequencing fragments containing such secondary structures can display increased electrophoretic mobility and "compression artifacts" which are manifested as compressed peaks (co-migrating bands) on CE electropherograms. Currently, urea is added to CE gels in an effort to avoid such compression artifacts and the resultant incomprehensible or erroneous results. This is only a partial solution, however, because urea is only soluble up to about 8M. Therefore, even with the maximum amount of urea added to such gels, compression artifacts are not completely eliminated.

Several approaches have been described for avoiding the compression artifact problem in slab gel electrophoresis. For example, use of nucleotide analogs for dideoxy sequencing has been successfully employed. However, such analogs are not effective in significantly reducing compression artifacts observed in CE gels. Heating of slab gels (e.g. to about 55°C) also is useful in preventing intramolecular base pairing of nucleic acids, but such heating can lead to rapid gel failure for CE gels. Thus the distinct differences between slab gel electrophoresis and CE means that solutions to the problems of slab gel electrophoresis are not always applicable to problems encountered with CE analytical methods.

Accordingly, a need exists for a capillary electrophoresis gel that overcomes the compression

artifact effect described above.

## SUMMARY OF THE INVENTION

The present invention satisfies this need. In accordance with the present disclosure, at least one neutral, non-urea denaturing agent comprising at least one moiety capable of disrupting hydrogen bonding and at least one moiety capable of reducing intramolecular base pairing is incorporated into the capillary electrophoresis gel formulation either singularly or in combination with urea. Most preferably, the moiety capable of disrupting hydrogen bonding is an amine ($NH_2$), and the moiety capable of reducing intramolecular base pairing is selected from the group consisting of H, an alkyl chain having from between about 1 to about 6 carbon atoms, a benzene ring, and substituted benzene rings. Amides, schematically represented as follows:

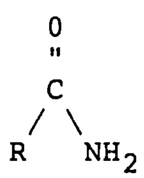

are particularly useful non-urea denaturants wherein R is the above described moiety capable of reducing intramolecular base pairing. Most preferably, R is hydrogen and thus the denaturing agent is formamide. When combined with urea, the gel formulation preferably comprises between about 6M and about 8M urea and between about 20% and 40% (by volume) of one or more of the non-urea denaturing agents. More preferably, about 6M urea and between about 30% and about 40% formamide, is utilized. When used singularly, i.e. without urea, the gel formulation comprises up to about 98% of the non-urea denaturing agent and most preferably up to about 50% of the non-urea denaturing agent.

These and other aspects of the invention will be described in detail below.

## BRIEF DESCRIPTION OF THE DRAWINGS

Fig. 1 is an electropherogram of a DNA sequence analyzed by capillary electrophoresis using a capillary gel containing only urea; and

Fig. 2 is an electropherogram of the same DNA sequence using a capillary gel in accordance with the present invention.

## DETAILED DESCRIPTION OF PREFERRED EMBODIMENTS

In accordance with the present disclosure, at least one neutral, non-urea denaturing agent comprising at least one moiety capable of disrupting hydrogen bonding and at least one moiety capable of reducing intramolecular base pairing is incorporated into the capillary electrophoresis gel formulation either singularly or in combination with urea. Most preferably, the moiety capable of disrupting hydrogen bonding is an amine ($NH_2$) and the moiety capable of reducing intramolecular base pairing is selected from the group consisting of H, an alkyl chain having from between about 1 to about 6 carbon atoms, a benzene ring, and substituted benzene rings. Amides, schematically represented as follows:

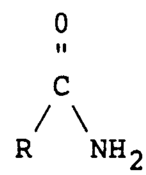

are particularly useful non-urea denaturants wherein R is the above described moiety capable of reducing intramolecular base pairing. Most preferably, R is hydrogen and the resultant amide is formamide.

When combined with urea, the gel formulation preferably comprises between about 6M and about 8M urea and between about 20% and 40% (by volume) of the non-urea denaturing agent. More preferably, about 6M urea and between about 30% and about 40% of the non-urea denaturant is utilized. When used singularly, i.e. without urea, the gel formulation comprises up to about 98% of the non-urea denaturing agent and most preferably up to about 50% of the non-urea denaturing agent.

The denaturing agent is advantageously electrically neutral in order to minimize conductivity of the gel and the deleterious effects associated therewith.

Amides of the above formulas are preferred because they include an $NH_2$ moiety which disrupts hydrogen binding and the R moiety interferes with nucleic acid base pairing which results in compression artifacts. Although not wishing to be bound thereto, Applicants believe that urea, an amide where R is $NH_2$, can effectively disrupt hydrogen bonding, but not base pairing. When R is as defined above a stronger denaturant (relative to urea) is produced, presumably through its effects on base pairing. Additionally, increasing the hydrophobicity by, for example, utilizing an alkyl chain having between about 1 to about 6 carbon atoms, benzene or benzene derivatives, also promotes more disruption of base pairing.

The non-urea denaturing agents must be co-soluble with the gel matrix or soluble in solvents to which the gel matrix is incorporated. For example, formamide, a liquid, is co-soluble with the gel matrix, while acetamide, a non-liquid, is soluble in solvents to which the gel matrix can be added. The non-urea denaturing agents must not interfere with formation of the gel matrix.

CE gels for use in the analysis of nucleic acids are well known and will not be set forth in detail here. Preferably, the gel formulation is acrylamide-based. Most preferably, the gel formulation includes N,N' methylene-bis acrylamide having a monomer:crosslinker ratio of either 29:1 or 19:1, TBE buffer tris-(hydroxymethyl) aminomethane-borate, EDTA, a pH range of from about 8.0 to about 9.5, and sufficient deionized water for formation of the correct gel consistency. To this formulation is added either between about 6M to about 8M urea and at least one of the disclosed non-urea denaturing agent, or at least one of the disclosed non-urea denaturing agent without urea. When formamide is the non-urea denaturing agent, the gel formulation should be substantially free of polyethylene glycol in that polyethylene glycol tends to precipitate in the presence of formamide upon cooling. Prior to filtration and degassing of the gel formulation, it is preferred that a mixed bead resin be added thereto in order to remove charged species from the formulation. Furthermore, polymerization of the gel should take place at a constant temperature, preferably 4°C, in order to avoid bubble formation.

A most preferred CE gel useful in the analysis of nucleic acid sequences in accordance with the present invention is prepared as follows: 6M urea; 10% (by volume) of the aforementioned TBE buffer; 10% (by volume) of 40% acrylamide (29:1 N'N methylene-bis acrylamide) and 35% (by volume) formamide are brought to 10 ml with deionized water. The final pH of the gel is about 8.6. To the mixture is added .5g mixed bead resin for 30 minutes prior to filtration and degassing. The polymerizing solution is then poured into a capillary tube (75μm i.d.). The tube may be derivatized according to procedures known to those in the art to permit covalent attachment of the gel to the capillary wall. However, this is not a requirement. The poured tubes are then capped at both ends to prevent drying of the gel and then placed in a 4°C storage container and allowed to polymerize.

## EXAMPLES

The following examples are presented for illustration purposes only and are not intended to limit the scope of the invention, this disclosure, or the claims to follow.

### I. Capillary Electrophoresis Instrumentation

The CE instrument utilized for the Examples was a breadboard high performance capillary electrophoresis laser induced fluorescence (HPCE/LIF) system. The HPCE/LIF system consisted of a plexiglass box enclosing two 4ml buffer vials, connected to a high voltage power supply (Bertan Associates, Inc., Model 205A-30R) by platinum wires submersed in each of the buffer vials. The power supply provides the voltage necessary for electrophoresis. The power supply was operated in the negative polarity configuration with the outlet end of the capillary maintained at ground potential. The current passing through the gel-filled capillaries was measured as a potential drop across a 1-KΩ resistor placed in the ground side of the circuit. A 2mm length of the protective polyimide coating was removed from the surface of the fused silica capillary using a thermal wire stripper (Western Electric Products Co., San Clemente, CA., Model G) equipped with blade elements. The 2mm section of the capillary was supported between swagelok fittings. The end of the separation capillary was dipped in the two buffer vials, each of which contained about 31ml of a CE buffer solution.

Sample introduction was accomplished by inserting the inlet end of the separation capillary into a microfuge tube containing the sample mixture and a short strip of platinum wire for electrical contact to high voltage. High voltage (about 15kV) was applied for about 10 seconds. Providing a means for electrical contact in the microfuge tube allows for sample introduction from the same sample tube in which the sequencing reactions were performed. This eliminated the need to pipette small sample volumes into an injection vial.

Detection was based upon laser-induced fluorescence using either the 448nm or the 514.5nm line of an air-cooled argon ion laser (Omichron, Chino, CA. Product No. 532AP). The laser light (approximately 8mW) was directed into the capillary separation channel using a 50 micron core optical fiber (Polymicro Technologies, Inc., Product No. FHA 050070125). The fluorescent emission was collected by the microscope objective was imaged onto a rectangular aperture which was sized to block the laser scatter originating from the interface between the capillary and the separation medium while passing most of the collected fluorescence. The fluorescent emission was then directed through two interference filters (550nm, 10nm band pass, Bar Associates, Inc., Westford MA., Product No. Custom S Cavity) and onto the photocathode of an end-on photomultiplier tube ("PMT") (Hamamatsu, San Jose, CA. Product No. R2228). PMT output was converted to voltage and electronically filtered (risetime = 100msec) using an in-house constructed preamp/filter unit. The

analog signal was digitized at $10H_z$ using a Macintosh 11X™ computer equipped with a MacArios 11/16th™ data acquisition board and Superscope™ software package (GW Instruments).

II. **DNA Fragments**

DNA sequencing reactions were performed using the "JOE"-labelled primer (Applied Biosystems, Inc., Foster City, CA., Product No. 400836). 1 µg of M13mp18 DNA (Pharmacia, Pistataway, NJ, Product No. 27-1516-01) was mixed with 0.5 picomole of the aforementioned primer and 2µL of 5X™ reaction buffer (Sequenase™ Polymerase kit, USB, Cleveland, Ohio, Product No. 70775) in a total volume of 1 µL. This was heated to 65°C, allowed to cool to 37°C, and 1µ of DTT solution and 1µL of Mn++ buffer (from the aforementioned Sequenase™ kit) were then added thereto. Thereafter, 9µL of a prewarmed termination mix was then added, followed by 2µL of undiluted Sequenase™ polymerase. The termination mix consisted of 500µM of each deoxynucleotide and 10µM of one dideoxy nucleotide, or was prepared by mixing 8.0µL, 4.0µL, and 2.0µL of similar mixtures, each containing different dideoxy nucleotides. The reaction was incubated for 30 minutes at 37°C after which it was placed on ice, followed by addition of 2µL of 3.0M sodium acetate, followed by 60µL of ice-cold ethanol. The DNA was pelleted by centrifugation at 12,000 RPM for 15 min. and rinsed once with 70% ethanol. The pellet was then dried and resuspended in 2.0µL of 80% formamide, 10mM EDTA. The sample was stored at -20°C in the dark and heated to 95°C for 1 minute just prior to injection onto the capillary.

III. **Capillary Gels**

10 ml of a capillary gel was prepared by admixing 4.2g urea (7M), 1 ml TBE 10X Buffer (Amresco, Salem, Ohio Product No. P0065841) and 1 ml of 40% 29:1 N,N'methylene-bis acrylamide (Amresco, Product No. P006584), to 10ml deionized water. The gel was designated "7M urea". 10 ml of a second gel was similarly prepared, the exceptions being that 3.5ml of deionized formamide (Amresco, Product No. 10060654) and urea (6M) were utilized. The gel was designated "6M urea, 35% formamide".

Approximately 30 minutes prior to filtration, .5g of mixed bead resin was added to the gels, followed by filtration and degassing. Capillaries (75 µm i.d.) were poured with the polymerizing material in accordance with standard procedures and stored at 4°C during polymerization.

**Example 1**

**Analysis on 7M Urea Gel**

Analysis of the described DNA sequence (having ddA:ddG:ddC ratio of 4:2:1) was performed on the described CE breadboard utilizing the 7M urea gel. The results thereof, set forth in the electropherogram depicted in Fig. 1, indicate that the peaks under the bar are compressed because 3-4 sequencing fragments appear as a single peak.

**Example 2**

**Analysis on 6M Urea, 35% Formamide Gel**

Analysis of the same DNA sequence on the CE breadboard utilizing 6M urea, 35% formamide gel provided significantly improved resolutions compared to the results of Example 1. The results, set forth in the electropherogram depicted in Fig. 2, show that the compression artifacts of Fig. 1 were significantly diminished.

The results from Examples 1 and 2 establish that the use of at least one non-urea denaturing agent in the gel matrix material significantly reduces the compression artifacts encountered when only urea is added to the gel.

The above examples are of preferred embodiments of the described invention. Modifications that are within the purview of those skilled in the art are intended to be within the scope of the invention.

**Claims**

1. A gel useful in capillary electrophoresis analysis of nucleic acids comprising:
   a) a gel material capable of being utilized in the capillary electrophoretic analysis of nucleic acids, and
   b) at least one neutral, non-urea, denaturing agent comprising at least one moiety capable of disrupting hydrogen bonding and at least one moiety capable of reducing intramolecular base pairing of nucleic acids.

2. The gel of claim 1 wherein the gel material further comprises between about 6M and about 8M urea.

3. The gel of claim 2 wherein the percent by volume of non-urea denaturing agent in the gel is between about 20% and about 40%.

4. The gel of claim 2 wherein the percent by volume of denaturing agent in the gel is between about 30% and about 40%.

5. The gel of claim 2 wherein the percent by volume of non-urea denaturing agent in the gel

is about 35%.

**6.** The gel of claim 1 wherein said non-urea denaturing agent is a compound represented by the formula:

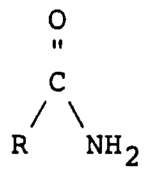

where R is selected from the group consisting of H, an alkyl chain having from about 1 to about 6 carbon atoms, benzene, and benzene derivatives.

**7.** The gel of claim 2 wherein said non-urea denaturing agent is formamide.

**8.** The gel of claim 1 wherein said non-urea denaturing agent is formamide.

**9.** The gel of claim 1 wherein the percent by volume of non-urea denaturing agent in the gel is less than about 98%.

**10.** The gel of claim 1 wherein the percent by volume of non-urea denaturing agent is less than about 50%.

**11.** A method for analyzing at least one nucleic acid sequence by capillary electrophoresis comprising the steps of:

a) obtaining a nucleic acid sequence to be analyzed;

b) loading said sequence onto a capillary gel having therein at least one neutral non-urea denaturing agent comprising at least one moiety capable of disrupting hydrogen bonding and at least one moiety capable of reducing intramolecular base pairing of nucleic acids;

c) subjecting said loaded sequence to capillary electrophoretic analysis; and

d) analyzing the results obtained by said capillary electrophoretic analysis.

**12.** The method of claim 11 wherein said moiety capable of disrupting hydrogen bonding is an amine.

**13.** The method of claim 11 wherein said moiety capable of reducing intramolecular base pairing is selected from the group consisting of hydrogen, an alkyl chain having from between about 1 to about 6 carbon atoms, benzene and benzene derivatives.

**14.** The gel of claim 13 wherein the gel material further comprises between about 6M and about 8M urea.

**15.** The gel of claim 13 wherein said non-urea denaturing agent is a compound represented by the formula:

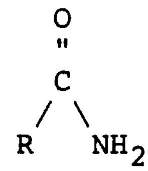

where R is selected from the group consisting of H, an alkyl chain having from about 1 to about 6 carbon atoms, benzene, and benzene derivatives.

**16.** The gel of claim 14 wherein said non-urea denaturing agent is a compound represented by the formula:

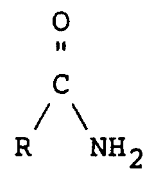

where R is selected from the group consisting of H, an alkyl chain having from about 1 to about 6 carbon atoms, benzene and benzene derivatives.

**17.** The gel of claim 15 wherein R is H, and said non-urea denaturing agent in said gel is present in amount less than about 98% by volume.

**18.** The gel of claim 16 wherein R is H, and said non-urea denaturing agent in said gel is present in an amount between about 20% and about 40% by volume.

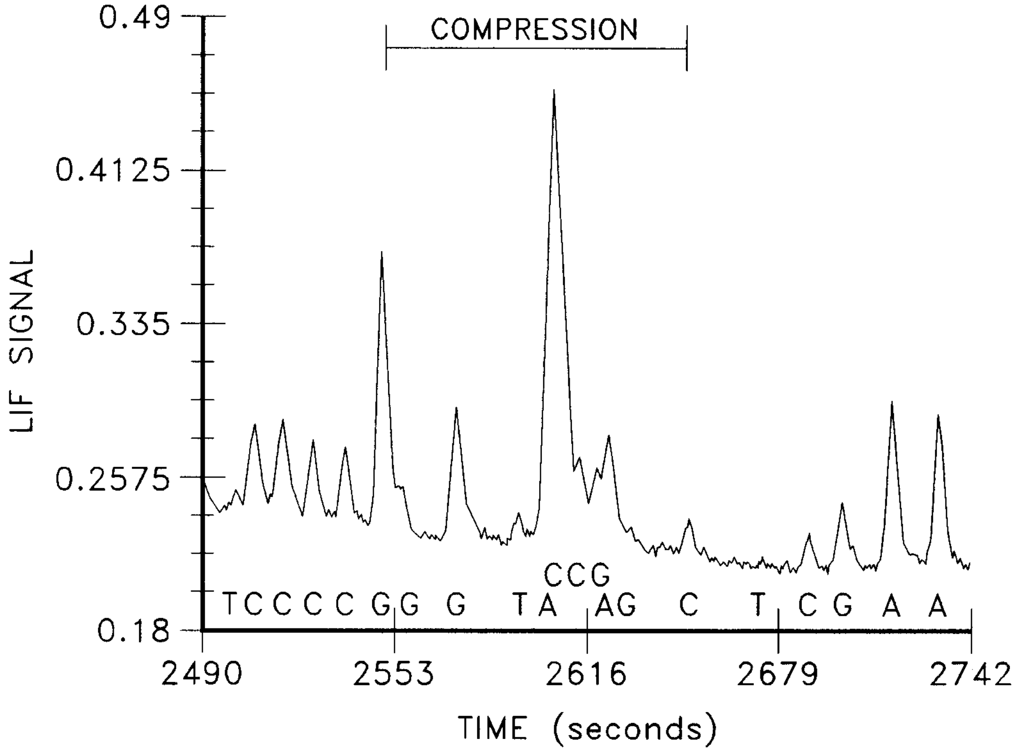
COMPRESSION
0.49
0.4125
0.335
0.2575
0.18
LIF SIGNAL
CCG
TCCCCGGG TA AG C T CGAA
2490
2553
2616
2679
2742
TIME (seconds)

FIG. 1

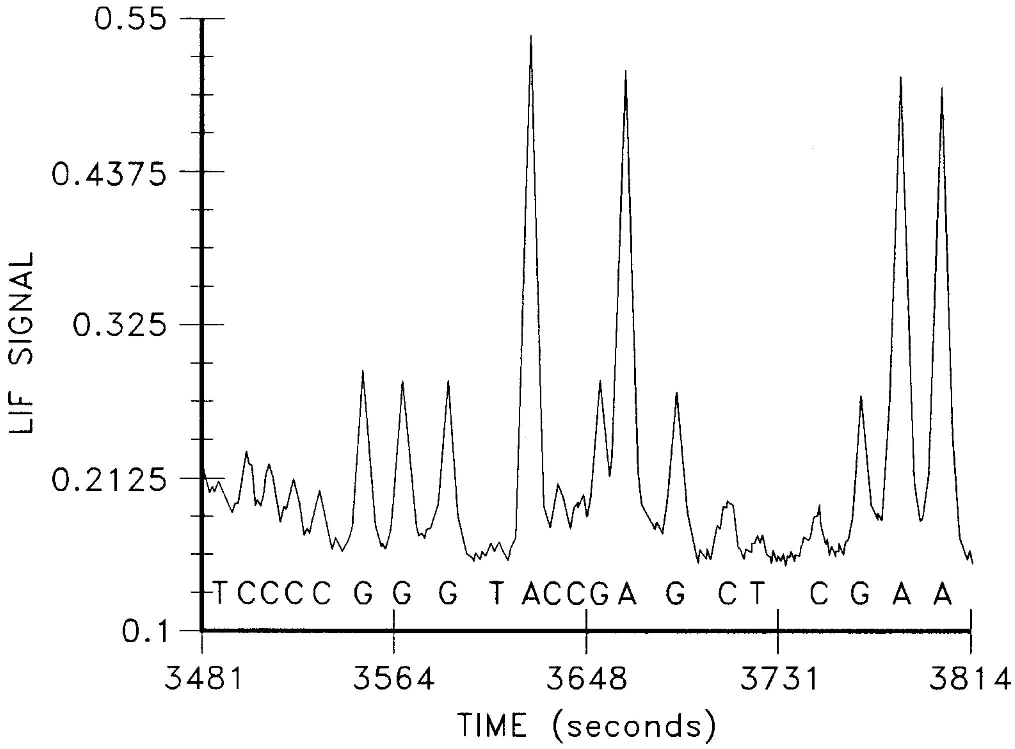
0.55
0.4375
0.325
0.2125
0.1
LIF SIGNAL
TCCCC G G G T ACCGA G CT C G A A
3481
3564
3648
3731
3814
TIME (seconds)

FIG. 2

European Patent Office

# EUROPEAN SEARCH REPORT

Application Number

EP 92 30 0077
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | WO-A-9 014 201 (SOANE D.S.)<br><br>* page 5, line 25 - line 33 *<br>* page 12, line 29 - page 13, line 10 *<br>* page 16, line 1 - line 18; claims 9,16 *<br>--- | 1,6,8,9, 11 | C12Q1/68<br>G01N27/26 |
| Y | NUCLEIC ACIDS RESEARCH.<br>vol. 9, 1981, ARLINGTON, VIRGINIA US<br>pages 4967 - 4979;<br>FRANK R. ET AL.: 'Identification and suppression of secondary structures formed from deoxyoligonucleotides during electrophoresis in denaturing polyacrylamide gels'<br>* abstract *<br>* page 4970, line 7 - line 19 *<br>* page 4978, line 2 - line 26 *<br>--- | 1,6,8,9, 11 | |
| A | CHROMATOGRAPHIA<br>vol. 24, 1987, BRAUNSCHWEIG<br>pages 15 - 24;<br>COHEN A.S. ET AL.: 'High-performance capillary electrophoresis using open tubes and gels'<br>* page 22, right column, line 7 - line 16; figure 8; table 1 *<br>--- | 1,11 | **TECHNICAL FIELDS SEARCHED (Int. Cl.5 )**<br><br>C12Q<br>G01N |
| A | US-A-4 699 705 (MASASHI OGAWA ET AL.)<br>* abstract *<br>* column 4, line 34 - line 45; claim 8 *<br>--- | 1,11 | |
| A | WO-A-8 902 930 (BOERRESEN A.L.)<br>* page 4, line 16 - line 30; claim 3 *<br>--- | 1-5,7 | |
| P,X | AMERICAN BIOTECHNOLOGY LABORATORY<br>vol. 9, no. 4, 1991, SHELTON,CT,USA<br>page 10;<br>A.GUTTMAN ET AL.: 'Denaturing capillary gel electrophoresis'<br>* The whole document, especially "Results" *<br><br>----- | 1,11 | |

The present search report has been drawn up for all claims

| Place of search | Date of completion of the search | Examiner |
|---|---|---|
| THE HAGUE | 20 MAY 1992 | LUZZATTO E.R. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P0401)